# EUROPÄISCHE PATENTSCHRIFT

(11) **EP 2 788 734 B1**
(45) Veröffentlichungstag und Bekanntmachung des Hinweises auf die Patenterteilung: **06.05.2020**
(21) Anmeldenummer: 12773276.6
(22) Anmeldetag: 27.09.2012
(51) Int. Cl.: G01N 3/40, G01N 33/15

(54) **REVOLVER FÜR TESTMASCHINE**
TURRET FOR A TEST MACHINE
TOURELLE REVOLVER POUR MACHINE D'ESSAI

(30) Priorität: 05.12.2011 DE 102011120070
(43) Veröffentlichungstag der Anmeldung: 15.10.2014
(73) Patentinhaber: Kraemer, Thilo, 64291 Darmstadt (DE)
(72) Erfinder: Kraemer, Thilo, 64291 Darmstadt (DE)
(74) Vertreter: Hamel, Armin
(86) Internationale Anmeldenummer: PCT/EP2012/069093
(87) Internationale Veröffentlichungsnummer: WO 2013/083308

(56) Entgegenhaltungen:
- EP-A2- 1 637 468
- DE-A1- 4 241 985
- DE-A1-102007 054 908
- US-A- 4 472 960
- US-A- 4 542 646
- US-A1- 2004 011 806

## Beschreibung

Die Erfindung betrifft eine Vorrichtung zur Durchführung eines Härtetests an Prüfkörpern mit einer Stützfläche zur Aufnahme des Prüfkörpers sowie einem Druckkolben und einem Gegenlager, wobei der Druckkolben und das Gegenlager oberhalb der Stützfläche angeordnet sind und mit einer Spindel, wobei der Druckkolben und das Gegenlager gegeneinander verfahrbar sind, wobei die Spindel den Druckkolben in Richtung Gegenlager bewegend ausgebildet ist, wobei sich der Prüfkörper im Anwendungsfall zwischen Druckkolben und Gegenlager befindet und die beim Gegeneinander drücken von Druckkolben und Gegenlager aufgewendete Kraft oder eine dazu proportionale Größe mittels einer Kraftmessvorrichtung messbar und die Härte des Prüfkörpers daraus bestimmbar ist.

Außerdem betrifft die Erfindung ein Verfahren zur Durchführung eines Härtetests an Prüfkörpern, wie Tabletten, Pillen, Oblongs, Dragees, Kapseln oder dergleichen auf einer Stützfläche, beispielsweise derjenigen eines Transportsterns, unter Verwendung einer zu schließenden und zu öffnenden Backenanordnung mit zwei einander gegenüberliegenden Backen, deren jede eine der anderen Backe zugewandte Kontaktfläche aufweist.

Als nächst kommender Stand der Technik wird von der europäischen Patentschrift EP 0 983 495 B1 ausgegangen. Die Patentschrift offenbart ein Verfahren und eine Vorrichtung zur Durchführung eines Härtetests an Prüfkörpern, insbesondere Tabletten oder Pillen.

US 4 542 646 beschreibt eine Prüfvorrichtung für Tabletten, bei der die Prüflinge einzeln zugeführt werden und die Druckachse senkrecht steht. DE 42 41 985 A1 beschreibt eine ähnliche Prüfvorrichtung bei der die Druckachse jedoch waagrecht liegt und die Prüflinge mit einer drehbaren Scheibe zugeführt werden. Die Drehachse dieser Scheibe ist jedoch senkrecht.

Dem Gegenstand der europäischen Patentschrift EP 0 983 495 B1 liegt die Aufgabe zu Grunde, ein Verfahren und eine Vorrichtung zur Durchführung eines Härtetests bei Prüfkörpern, insbesondere Tabletten oder Pillen, zu entwickeln, bei welcher das Ausrichten des Prüfkörpers, relativ zu Druckkolben und Gegenlager in kurzer Zeit mit wenig Rechenaufwand zu bewerkstelligen ist. Die Aufgabe wird dadurch gelöst, dass auf optischem, elektronischem oder akustischem Weg die räumliche Lage einer Vorzugsachse ermittelt wird, das Korrekturdaten ermittelt werden, die von diesen Lagedaten und von der Position von Druckkolben und Gegenlager abhängig sind und das Druckkolben und Gegenlager auf Grund dieser Korrekturdaten so relativ zum in seiner Lage auf dem Prüftisch unveränderten Prüfkörper verfahren werden, dass die Prüfrichtung, das heißt die Richtung des Gegeneinanderdrückens von Druckkolben und Gegenlager mit der Richtung der Vorzugsachse übereinstimmt und dass das Gegenlager in unmittelbarer Nähe des Prüfkörpers angebracht wird und das die Härte des Prüfkörpers daraufhin durch Herandrücken des Druckkolbens gegen den Prüfkörper bei unveränderter Position des Gegenlagers bestimmt wird.

Nachteilig am Stand der Technik ist der Prüftisch. Zum Positionieren des Prüfkörpers steht auf Grund des einen Tisches nur dessen Tischoberseite als eine Stützfläche zur Verfügung. Zum Belegen dieser einen Stützfläche ist diese entweder aus dem Bereich zwischen Druckkolben und Gegenlager heraus zu verfahren. Alternativ ist mittels Zuführvorrichtungen ein Prüfkörper auf die Stützfläche zu verbringen. Das Verschieben des Prüftisches zur Belegung mit einem Prüfkörper oder das Belegen des Prüftisches mit Prüfkörpern mittels Schiebern ist zeitaufwendig.

Aufgabe der Erfindung ist, ein Zuführen und Ausrichten eines Prüfkörpers in kürzester Zeit und mit minimalstem Rechenaufwand zu bewerkstelligen.

Diese Aufgabe wird bei einer Vorrichtung zur Durchführung eines Härtetests an Prüfkörpern mit einer Stützfläche zur Aufnahme des Prüfkörpers sowie einem Druckkolben und einem Gegenlager, die oberhalb der Stützfläche angeordnet sind und mit einer Spindel, wobei der Druckkolben und das Gegenlager gegeneinander verfahrbar sind, wobei die Spindel dem Druckkolben in Richtung Gegenlager bewegend ausgebildet ist, wobei sich der Prüfkörper im Anbindungsfall zwischen Druckkolben und Gegenlager befindet und die beim Gegeneinanderdrücken von Druckkolben und Gegenlager aufgewendete Kraft oder eine dazu proportionale Größe mittels einer Kraftmessvorrichtung messbar und die Härte des Prüfkörpers daraus bestimmbar ist, dadurch gelöst, dass die Stützfläche als eine Aussparung eines Transportsterns ausgebildet ist und die Drehachse des Transportsterns horizontal parallel zu der Spindelachse angeordnet ist.
[A1] Wünschenswerterweise werden Anhaftungen an den Frontflächen eines am vorderen Ende des Druckkolbens angeordneten Stößels und an der Frontfläche des Gegenlagers durch die Kanten des Transportsterns abgeschert. Die Vorrichtung ist damit selbstreinigend. Wenn der Transportstern um einen Rotationswinkel gedreht wird, scheren seine Kanten entlang der Frontflächen von Stößel und Gegenlager mit. Der Transportstern ist ein Rotationsmagazin. Rotationsmagazine arbeiten schnell und sind unkompliziert im Handling. Wenn die Stützfläche als eine Aussparung eines Transportsterns in einem Rotationsmagazin ausgebildet ist, ist der Weg zum Nachführen neuer Prüfkörper minimiert. Die Zeit zum Nachführen von Prüfkörpern ist minimiert. Die Messstrecke zur Tablettenprüfung liegt zwischen Druckkolben und Gegenlager. Beim Verlassen der Stützfläche aus der Messstrecke wird direkt eine neue Stützfläche nachgeführt. Dadurch, dass die Drehachse des Transportsterns vertikal parallel zu der Spindelachse angeordnet ist, kann die Drehachse des Transportsterns unterhalb der Spindelachse angeordnet werden. Somit ist es möglich den Prüfkörper in einer Aufwärtsbewegung in die Messstrecke nachzuführen. Durch den vertikalen Versatz der Drehachse gegenüber der Spindelachse sind kleinere Umfängen des Transportsterns umsetzbar, so dass mit kleinerem Umfang kürzere Zeiten zum Nachführen der Prüfkörper umsetzbar sind. Transportsterne in Form von Revolvertrommel sind kostengünstig aus dem Vollen fräsbar. Dadurch, dass Aussparungen in Form von Rund- oder eckigen Nuten vorgesehen sind, sind Prüfkörper unkompliziert in die Aussparung zu schütten. Wünschenswerterweise sind Vorrichtungen zum zügigen Wechsel von Transportsternen bekannt, so dass für unterschiedliche Prüfkörpergrößen Transportsterne durch Wechsel für den Fachmann einfach umsetzbar sind.
[A2] In einer bevorzugten Ausführungsform ist das Profil der mindestens einen Aussparung des Transportsterns mit dem Profil eines an einem Ende des Druckkolbens montierten Stößels mindestens teilweise ergänzt und ausgebildet. Dadurch, dass sich die Profile ergänzen, sind die Spaltmaße zwischen Stößel und Aussparung eng toleriert, so dass der Prüfkörper während des Messvorganges entlang der Transportsternbreite durch die Aussparung klemmfrei geführt wird.
   Ein Verklemmen, Durchrutschen oder Zerbröseln des Prüfkörpers wird vermieden. Die Ergänzung der Profile bildet somit eine redundante Lagekontrolle des Transportsterns, denn nur, wenn der Stößel reibungsfrei bis zum Beginn der Bruchmessung läuft, bleibt ein Messsignal aus.
[A3] Dadurch, dass das Profil des Transportsterns konkav ausgebildet ist, zentrieren sich rundförmige Prüfkörper in der Aussparung selbstständig. In die Aussparung zugeführte Prüfkörper rutschen in den Wendepunkt des konkaven Profils, so dass ihre Ausrichtung und Position bekannt ist. Konkave Profile sind einfach herstellbar und weisen weder Ecken noch Kanten auf in denen Rückstände verbleiben können. Konkave Profile ermöglichen ein Ab- und Ausrollen von Prüfkörpern. Die Zerstörung von Prüfkörpern durch ein Aufschlagen der Prüfkörper gegen Kanten wird somit verhindert.
[A4] Sinnvollerweise sind die Aussparungen radial offen als Halbschalen ausgebildet. Offene Halbschalen sind einfach zu befüllen und entleeren sich mit der Schwerkraft selbstständig.
[A5] Optimalerweise weist der Transportstern mindestens sechs Aussparungen auf. In fünf von sechs Aussparungen können während der Messung verschiedene Arbeitsgänge ausgeführt werden. Während der sechsten Aussparung die Zuführung, das Entleeren und das Reinigen vorgenommen werden.
[A6] In einer bevorzugten Ausführungsform ist eine Reinigungsvorrichtung zur Reinigung der Aussparung vorgesehen. Nach dem Bruch eines Prüfkörpers sind Rückstände in der Aussparung damit beseitigbar, so dass eine gereinigte Aussparung für die Vermessung eines weiteren Prüfkörpers zur Verfügung steht. Fehler in der Messstrecke durch Verunreinigungen werden damit minimiert. Die Reinigung von Aussparungen ermöglicht ein Vermessen von unterschiedlichen Prüfkörpern im Betrieb der Vorrichtung.
[A7] Die Aufgabe wird ferner durch ein Verfahren zur Prüfung eines Härtetests an Prüfkörpern, wie Tabletten, Pillen, Oblongs, Dragees, Kapseln oder der gleichen, auf einer Stützfläche, beispielsweise derjenigen eines Transportsterns, unter Verwendung einer zu schließenden und zu öffnenden Backenanordnung mit zwei einander gegenüberliegenden Backen, deren jede eine der jeweils anderen Backe zugewandte Kontakt aufweist, dadurch gelöst, das der Prüfkörper teilweise auf einem kreisförmigen Weg in einer vertikalen Ebene bewegt wird, vorzugsweise intermittierend. Mittels der kreisförmigen Wegführung wird die Gravitation auf dem Prüfkörper ausgenutzt. Die Gravitation unterstützt und ermöglicht ein Schütten von Prüfkörpern in die Aussparung eines Transportsterns und ein selbstständiges Leeren der Aussparungen. Eine rotatorische Bewegung ist sehr genau steuerbar. Der Transportstern ist ohne Getriebe an einem Digitalservomoter anschließbar.
[A8] Dadurch, dass an den Backen anhaftende Reste, vorzugsweise Krümel oder Bruchstücke durch Kanten des Transportsterns bei dessen Drehung entfernt werden, sind die Backen stets gereinigt. Dadurch, dass die Frontflächen der Backen gereinigt sind, treffen sie vertikal auf die Enden des Prüfkörpers und bewegen diesen ohne seitliches Verrutschen, so dass eine Falschpositionierung des Prüfkörpers beim Schließen der Backenanordnung verhindert wird.
[A9] Wenn durch Vibration des Transportsterns der Prüfkörper positioniert und/oder ausgerichtet wird, wird er in die richtige Position geschüttelt.
[A10] Sinnvollerweise werden die Stützflächen des Transportsterns mittels einer Bürste oder eines Wasserstrahls oder eines Pressluftstrahls gereinigt.

Eine bevorzugte Ausführungsform der Erfindung wird beispielhaft anhand einer Zeichnung erläutert. Die Figurenbezeichnung zeigen im Einzelnen:
- Figur 1: eine perspektivische Ansicht einer Vorrichtung zur Durchführung eines Härtetest an Prüfkörpern mit einer Revolvertrommel als Transportstern,
- Figur 2: eine vergrößerte perspektivische Darstellung eines Transportsterns,
- Figur 3: eine Seitenansicht auf den Transportstern aus Figur 2,
- Figur 4: eine Aufsicht den Transportstern aus Figur 2 und 3,
- Figur 5: eine schematische Darstellung des Transportsterns,
- Figur 6: eine schematische Darstellung des Transportsterns in einer perspektivischen Ansicht,
- Figur 7: eine schematische Ansicht des Transportsterns von der Seite und
- Figur 8: einen Ausbruch aus einer Vorderansicht einer Vorrichtung zur Durchführung eines Härtetests,
- Figur 9: eine Prinzipskizze des Standes der Technik.

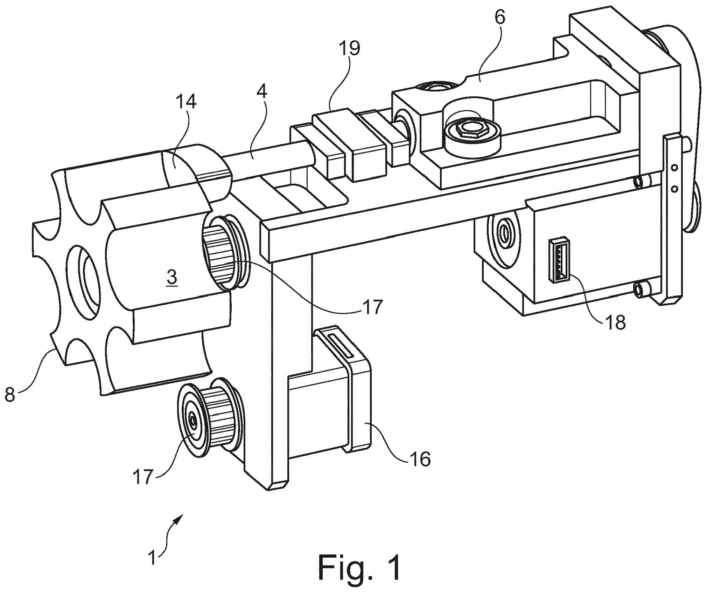

In Figur 1-7 bezeichnet die Bezugsziffer 1 die erfindungsgemäße Vorrichtung zur Durchführung eines Härtetests an Prüfkörpern.

In Figur 1 ist eine Vorrichtung zur Durchführung eines Härtetests 1 in perspektivischer Ansicht gezeigt. Die Vorrichtung setzt sich zusammen aus einem Prüftisch, welcher die Antriebsspindel 6 mit dem Spindelmotor 18 trägt. Ferner haltert der Prüftisch die Zahnräder für einen Zahnriemenantrieb 17, welcher die Rotation des Revolverantriebs 16 auf den Transportstern 8 überträgt. Der Zahnriemen ist hier nicht gezeigt. Mit der Spindel 16 ist die Kraftmessdose 19 verschraubt. Auf der gegenüberliegenden Seite der Kraftmessdose 19 ist diese mit dem Druckkolben 4 verschraubt. Der Druckkolben 4 ist mit einem Stößel 14 verschraubt. Durch den Spindelmotor 18 wird die Spindel 6 angetrieben, so dass diese die Rotation des Spindelmotors 18 in eine Linearbewegung von Druckkolben und Stößel übersetzt. Der durch die Linearbewegung erzeugte Vorschub, leitet eine Kraft in den Prüfkörper 2 ein. Der Prüfkörper 2 liegt dafür in einer der Aussparungen 9 auf der Stützfläche 3. Die Stützfläche 3 ist die Innenfläche der Aussparung 9 des Transportsterns 8. Der Prüfkörper 2 wird durch die eingeleitete Kraft des Stößels 14 gegen die Frontfläche des Gegenlagers 5 gepresst und leitet die Kraft in das Gegenlager 5 ein, bis der Prüfkörper 2 birst. Das Gegenlager 5 ist in dieser Ansicht nicht dargestellt, es leitet die Kraft in den Rahmen der Vorrichtung.

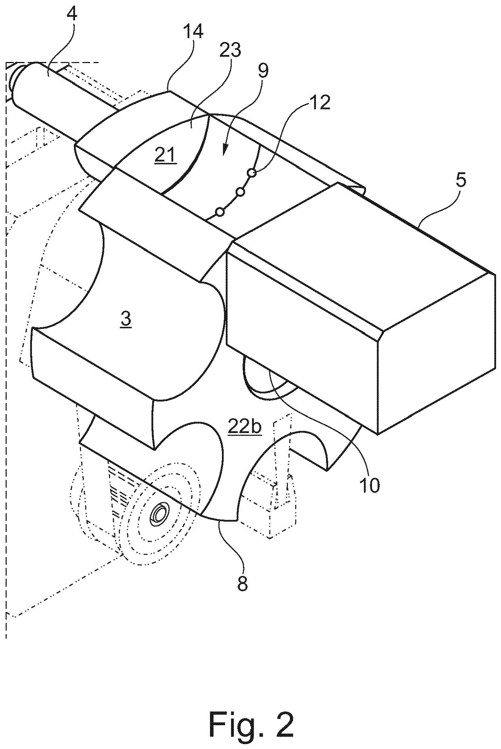

Figur 2 zeigt perspektivisch einen Transportstern 8 in Form einer Revolvertrommel in der Anwendungslage. Gezeigt ist die Ausgangsposition des Stößels 14. Die Stoßfläche des Stößels 21 passt die ihr zugewandte Seitenfläche 22a des Transportsterns 8 auf Pressung. In der Ausgangslage kann der Transportstern 8 damit frei um seine Drehachse 11 rotieren. Auf Grund der Presspassung zwischen der Stoßfläche des Stößels 21 und der Seitenfläche des Transportsterns 22b, werden Pressreste 23 von der Stößelfläche 21 abgeschert. Das Gegenlager 5 ist fixiert.

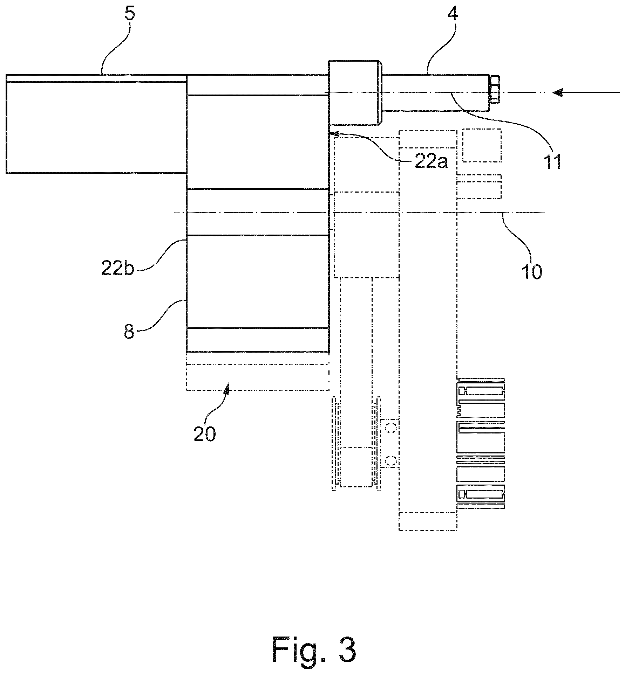

In Figur 3 ist eine Seitenansicht auf den Transportstern 8 gezeigt. Das Gegenlager 5 ist auf Preessung mit der Seite 22b des Transportsterns 8 gepasst. Somit werden ebenfalls Pressreste 23 von der Stoßfläche des Gegenlagers 5 bei Rotation des Transportsterns 8 abgeschert. Figur 3 zeigt, dass die Drehachse 10 des Transportsterns 8 von der Spindelachse 11 beabstandet ist. Die Drehachse 10 des Transportsterns 8 und der Spindelachse 11 verlaufen vertikal parallel. Der Abstand zwischen ihnen enrspricht dem Radius des Transportsterns 8.

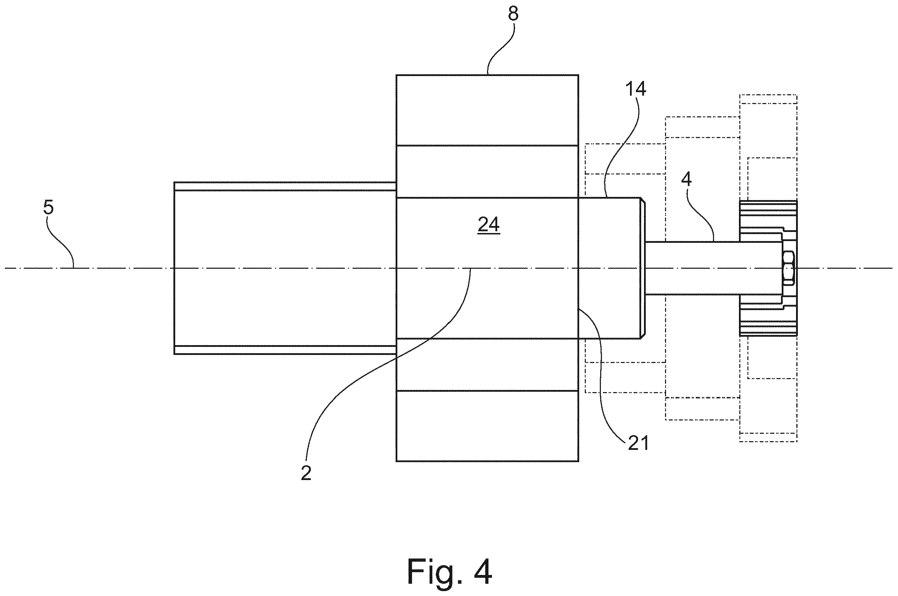

Figur 4 zeigt einen Transportstern 8 mit Gegenlager 5 und Druckkolben 4 in der Aufsicht. Zwischen dem Stößel 14 und dem Gegenlager 5 liegt die Bruchkammer 24. Durch den linearen Vorschub des Stößels 14 in Richtung des Gegenlagers 5 wird eine in der Bruchkammer 24 liegende Tablette von dem Stößel 14 auf das Gegenlager 5 geschoben. Sobald die Stoßflächen des Stößels 21 die Tablette 2 gegen die Stoßfläche des Gegenlagers 5 verschoben hat, beginnt die Kraftmessung. Bei der Kraftmessung werden über die Kraftmessdose 19 in die Tablette eingeleiteten Kräfte vermessen.

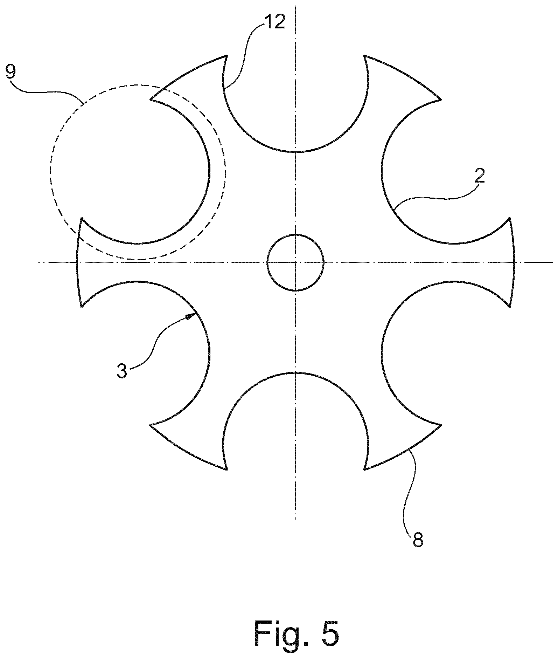

Figur 5 zeigt eine schematische Darstellung einer Revolvertrommel. Die Revolvertrommel zeigt Stützflächen 3 in den Aussparungen 9. Das Profil der Aussparungen 12 ist eine Halbschale.

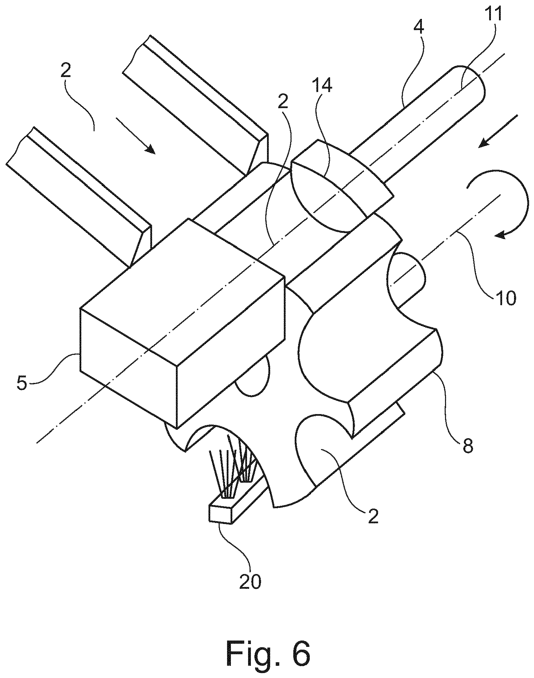

Figur 6 zeigt ebenfalls eine schematische Darstellung für die Zufuhr eines Prüflings 2 in die Vorrichtung zur Durchführung des Härtetestes. Der Prüfkörper 2 wird über eine Rampe oder eine Rutsche in eine der Aussparungen 9 des Transportsterns 8 zugeführt. Nach einer bestimmten Taktung mittels einer Computersteuerung, wird der Prüfling 2 durch Rotation des Transportsterns 8 zwischen dem Gegenlager 5 und dem Stößel 14 positioniert. Zwischen der nächsten Taktung kann nun der Stößel 14 zur Vermessung der Härte des Prüfkörpers 2 in die Ausparung 2 ein- und ausgefahren werden. Nach der Vermessung wird der Prüfling 2 durch die weitere Rotation des Transportsterns 8 aus der Prüflage herausgedreht. Dabei wird die Aussparung 9 auf Grund der Gravitationen von dem Bruchresten des Prüflings 2 befreit. Danach ist die Reinigung der Aussparungen 9 mittels einer Bürste 20 oder eines Wasserstrahls oder eines Luftstrahls möglich.

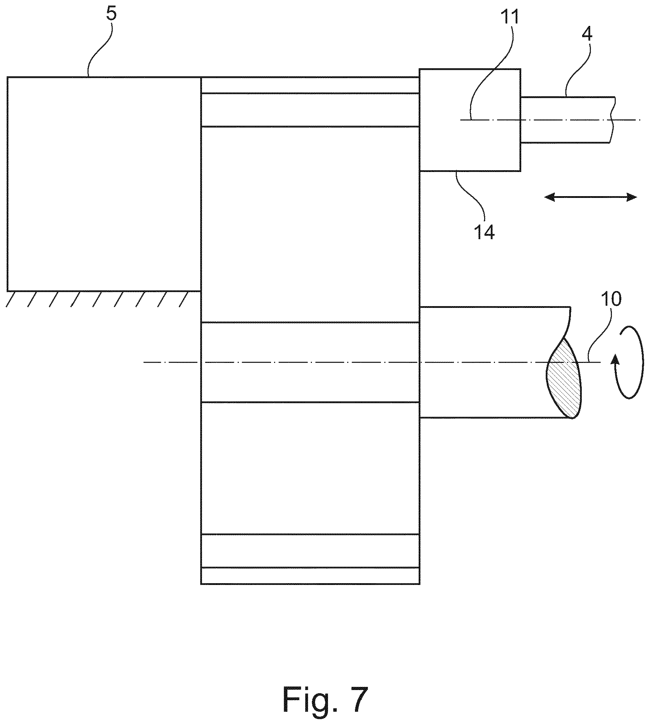

Figur 7 zeigt eine Seitenansicht der in Figur 6 dargestellten Transportes.

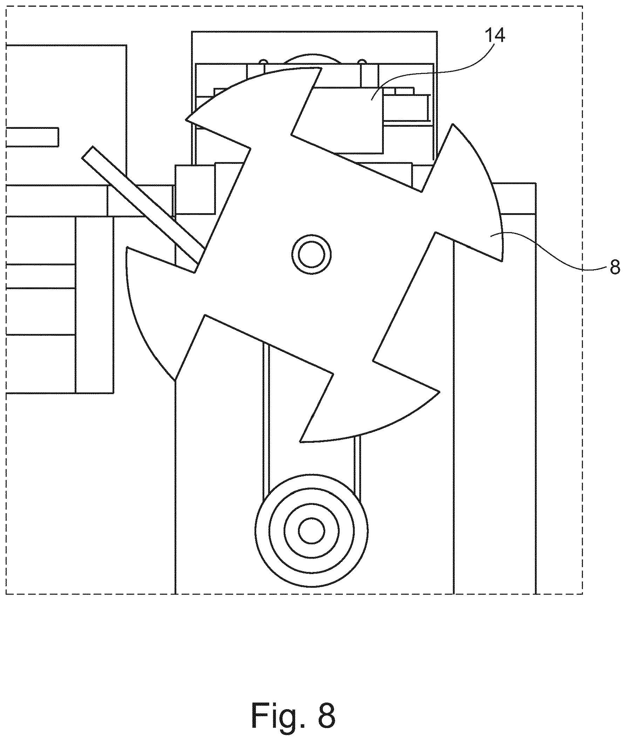

Figur 8 zeigt eine Aussparung 9 einer Frontalansicht einer Vorderansicht auf eine Vorrichtung zur Durchführung eines Härtetests.

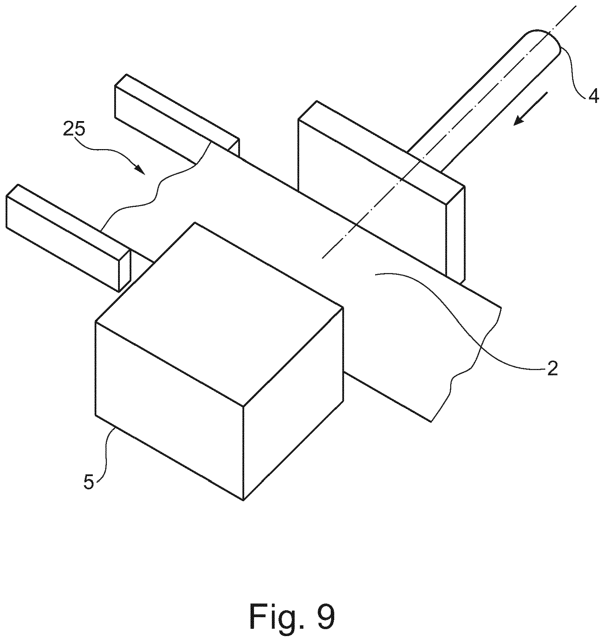

Figur 9 zeigt eine Bruchkammer in einer schematischen Darstellung zwischen einem Druckkolben 4 und einem Gegenlager 5. Entgegen dem erfinderischen Gegenstand wird eine Prüflingszufuhr 25 durch den seitlichen Eintrag gezeigt.

### Bezugsziffernliste

- 1.: Vorrichtung zur Durchführung eines Härtetest
- 2.: Prüfkörper
- 3.: Stützfläche
- 4.: Druckkolben
- 5.: Gegenlager
- 6.: Spindel
- 7.: Kraftmessvorrichtung, Kraftmessdose
- 8.: Transportstern
- 9.: Aussparung
- 10.: Drehachse
- 11.: Spindelachse
- 12.: Profil der Aussparung
- 13.: Profil des Stößels
- 14.: Stößel
- 15.: Reinigungsvorrichtung
- 16.: Revolverantrieb
- 17.: Zahnriemenantrieb
- 18.: Spindelmotor
- 19.: Kraftmessdose
- 20.: Reinigungsvorrichtung, Bürste
- 21.: Stoßfläche des Stößels
- 22: a,b Seitenfläche des Transportsterns
- 23.: Tablettenreste, Pressreste
- 24.: Bruchkammer
- 25.: Zufuhr

## Patentansprüche

1. Vorrichtung zur Durchführung eines Härtetests (1) an Prüfkörpern (2) mit einer Stützfläche (3) zur Aufnahme des Prüfkörpers (2) sowie einem Druckkolben (4) und einem Gegenlager (5), die oberhalb der Stützfläche (3) angeordnet sind und mit einer Spindel (6), wobei der Druckkolben (4) und das Gegenlager (5) gegeneinander verfahrbar sind, wobei die Spindel (6) den Druckkolben (4) in Richtung Gegenlager (5) bewegend ausgebildet ist, wobei sich der Prüfkörper (2) im Anwendungsfall zwischen Druckkolben (4) und Gegenlager (5) befindet und die beim Gegeneinanderdrücken von Druckkolben (4) und Gegenlager (5) aufgewendete Kraft oder eine dazu proportionale Größe mittels einer Kraftmessvorrichtung (7) messbar und die Härte des Prüfkörpers (2) daraus bestimmbar ist, **dadurch gekennzeichnet, dass** die Stützfläche (3) als eine Aussparung (9) eines Transportsterns (8) ausgebildet ist und die Drehachse (10) des Transportsterns (8) horizontal parallel zu der Spindelachse (11) angeordnet ist.

2. Vorrichtung nach Anspruch 1, **dadurch gekennzeichnet, dass** das Profil (12) der mindestens einen Aussparung (9) des Transportsterns (8) mit dem Profil (13) eines an einem Ende des Druckkolbens (4) montierten Stößels (14) mindestens teilweise ergänzend ausgebildet ist.

3. Vorrichtung nach Anspruch 1 oder 2, **dadurch gekennzeichnet, dass** das Profil (12) der Aussparung (9) konkav ausgebildet ist.

4. Vorrichtung nach Anspruch 1, 2 oder 3, **dadurch gekennzeichnet, dass** die Aussparung (9) radial offen als Halbschale ausgebildet sind.

5. Vorrichtung nach einem oder mehreren der vorhergehenden Ansprüche, **dadurch gekennzeichnet, dass** der Transportstern (8) mindestens eine Aussparung (9) aufweist.

6. Vorrichtung nach einem oder mehreren der vorhergehenden Ansprüche, **dadurch gekennzeichnet, dass** eine Reinigungsvorrichtung (15) zur Reinigung der Aussparungen (9) vorgesehen ist.

7. Verfahren zur Durchführung eines Härtetests an Prüfkörpern, wie Tablette, Pille, Oblong, Dragee, Kapsel oder dergleichen, auf einer Stützfläche, beispielsweise derjenigen eines Transportsterns, unter Verwendung einer zu schließenden und zu öffnenden Backenanordnung mit zwei einander gegenüberliegenden Backen, deren jede eine der jeweils anderen Backe zugewandte Kontaktfläche aufweist, **gekennzeichnet durch den** Schritt, **dass** der Prüfkörper teilweise auf einem kreisförmigen Weg in einer vertikalen Ebene bewegt wird, vorzugsweise intermittierend.

8. Verfahren nach Anspruch 7, **gekennzeichnet durch den Schritt**, **dass** an den Backen anhaftende Reste, vorzugsweise Krümel oder Bruchstücke durch Kanten der Seitenflächen des Transportsterns bei dessen Drehung entfernt werden.

9. Verfahren nach Anspruch 7 oder 8, **gekennzeichnet durch den Schritt**, dass durch Vibrationen des Transportsterns, der Prüfkörper positioniert und/oder ausgerichtet wird.

10. Verfahren nach Anspruch 7, 8 oder 9, **gekennzeichnet durch den Schritt**, dass die Stützflächen des Transportsterns mittels einer Bürste oder eines Wasserstrahls oder eines Pressluftstrahls gereinigt werden.

## Claims

1. Device for carrying out a hardness test (1) on test specimens (2), with a supporting surface (3) for receiving the test specimen (2) as well as a pressure piston (4) and a counter bearing (5), that are disposed above the supporting surface (3), and with a spindle (6), wherein the pressure piston (4) and the counter bearing (5) are movable against one another, wherein the spindle (6) is implemented such that it moves the pressure piston (4) in the direction toward the counter bearing (5), wherein the test specimen (2) in the application case is located between pressure piston (4) and counter bearing (5) and the force expended during the pressing of pressure piston (4) and counter bearing (5) against one another or a value proportional thereto is measurable by means of a force gauge (7) and the hardness of the test specimen (2) is determinable therefrom, **characterized in that** the supporting surface (3) is developed as a recess (9) of a transport star wheel element (8) and the axis of rotation (10) of the transport star wheel element (8) is disposed horizontally in parallel with the spindle axis (11).

2. Device as in claim 1, **characterized in that** the profile (12) of the at least one recess (9) of the transport star wheel element (8) is developed such that it at least partially complements the profile (13) of a tappet (14) mounted at an end of the pressure piston (4).

3. Device as in Claim 1 or 2, **characterized in that** the profile (12) of the recess (9) is developed in a concave shape.

4. Device as in Claim 1, 2 or 3, **characterized in that** the recess (9) are [*sic* : is] developed in the form of a radially open half-shell.

5. Device as in one or more of the preceding claims, **characterized in that** the transport star wheel element (8) comprises at least one recess (9).

6. Device as in one or more of the preceding claims, **characterized in that** a cleaning fixture (15) is provided for cleaning the recesses (9).

7. Method for carrying out a hardness test on test specimens, such as tablets, pills, oblongs, dragees, capsules or the like, on a supporting surface [3], for example that of a transport star wheel element [8], utilizing a jaw configuration to be closed and opened with two opposing jaws, each of which comprising a contact face facing the in each instance other jaw, **characterized by the step that** the test specimen [2] is partially moved, preferably intermittently, on a circular path in a vertical plane.

8. Method as in claim 7, **characterized by the step that** residues, preferably debris or fragments, adhering on the jaws are removed by tilting the side faces of the transport star wheel element [8] during its rotation.

9. Method as in claim 7 or 8, **characterized by the step that** the test specimen [2] is positioned and/or oriented through vibrations of the transport star wheel element [8].

10. Method as in claim 7, 8 or 9, **characterized by the step** that the support surfaces [3] of the transport star wheel element [8] are cleaned by means of a brush or a water jet or a compressed air jet.

## Revendications

1. Dispositif destiné à effectuer un test de dureté (1) sur des corps à tester (2), ledit dispositif comprenant une surface de support (3) destinée à recevoir le corps à tester (2) ainsi qu'un piston de pression (4) et une butée (5), qui sont disposés au-dessus de la surface de support (3), et une broche (6), le piston de pression (4) et la butée (5) pouvant être déplacés l'un par rapport à l'autre, la broche (6) étant conçue pour déplacer le piston de pression (4) en direction de la butée (5), le corps à tester (2) étant situé dans le cas d'application entre le piston de pression (4) et la butée (5) et la force appliquée lorsque le piston de pression (4) et la butée (5) sont pressés l'un contre l'autre ou une grandeur proportionnelle à celle-ci pouvant être mesurée au moyen d'un dispositif de mesure de force (7) et la dureté du corps à tester (2) pouvant être déterminée à partir de là, **caractérisé en ce que** la surface de support (3) est conçue comme un évidement (9) d'une étoile de transport (8) et l'axe de rotation (10) de l'étoile de transport (8) est disposé horizontalement et parallèlement à l'axe de broche (11).

2. Dispositif selon la revendication 1, **caractérisé en ce que** le profil (12) de l'au moins un évidement (9) de l'étoile de transport (8) est au moins partiellement complémentaire du profil (13) d'un piston (14) monté sur une extrémité du piston de pression (4).

3. Dispositif selon la revendication 1 ou 2, **caractérisé en ce que** le profil (12) de l'évidement (9) est concave.

4. Dispositif selon la revendication 1, 2 ou 3, **caractérisé en ce que** l'évidement (9) est conçu pour être ouvert radialement en demi-coque.

5. Dispositif selon l'une au moins des revendications précédentes, **caractérisé en ce que** l'étoile de transport (8) comporte au moins un évidement (9).

6. Dispositif selon l'une au moins des revendications précédentes, **caractérisé en ce qu'**un dispositif de nettoyage (15) est prévu pour nettoyer les évidements (9).

7. Procédé destiné à effectuer un test de dureté sur des corps à tester, tels que des comprimés, des pilules, des cachets oblongs, des dragées, des gélules ou similaires, sur une surface de support, par exemple celle d'une étoile de transport, à l'aide d'un ensemble de mâchoire à fermer et à ouvrir pourvu de deux mâchoires opposées qui comportent chacune une surface de contact faisant face à l'autre mâchoire, **caractérisé par l'étape** selon laquelle le corps à tester est déplacé partiellement sur un trajet circulaire dans un plan vertical, de préférence par intermittence.

8. Procédé selon la revendication 7, **caractérisé par l'étape** selon laquelle des résidus adhérant aux mâchoires, de préférence des débris ou des fragments, sont éliminés par des bords des faces latérales de l'étoile de transport lors de la rotation de celle-ci.

9. Procédé selon la revendication 7 ou 8, **caractérisé par l'étape** selon laquelle le corps à tester est positionné et/ou orienté par des vibrations de l'étoile de transport.

10. Procédé selon la revendication 7, 8 ou 9, **caractérisé par l'étape** selon laquelle les surfaces de support de l'étoile de transport sont nettoyées au moyen d'une brosse ou d'un jet d'eau ou d'un jet d'air comprimé.
